# EUROPEAN PATENT SPECIFICATION

(11) **EP 0 366 765 B1**
(45) Date of publication and mention of the grant of the patent: **30.11.1994**
(21) Application number: 89905513.1
(22) Date of filing: 26.04.1989
(51) Int. Cl.: A61K 31/135

(54) **ANTIGLAUCOMA COMPOSITIONS CONTAINING COMBINATIONS OF SYMPATHOMIMETIC AGENTS AND BETA-1 SELECTIVE BETA-BLOCKERS**
KOMBINATIONEN VON SYMPATHOMIMETIKA MIT BETA-1 SELEKTIVEN BETABLOCKERN ENTHALTENDE MITTEL GEGEN GLAUCOMA
COMPOSITIONS ANTIGLAUCOME CONTENANT DES COMBINAISONS D'AGENTS SYMPATOMIMETIQUES ET DE BETA-BLOQUANTS A SELECTION DE BETA-1

(30) Priority: 26.04.1988 US 186415
(43) Date of publication of application: 09.05.1990
(73) Proprietor: ALCON LABORATORIES, INC., Fort Worth Texas 76134-2099 (US)
(72) Inventor: DE SANTIS, Louis, M., Fort Worth, TX 76109 (US)
(74) Representative: Jump, Timothy John Simon
(86) International application number: US8901748
(87) International publication number: WO8910120

(56) References cited:
- US-A- 3 852 468
- Chemical Abstract, Volume 109, No. 7, issued 15 August 1988, B. Schwartz; see page 76, column 1
- Biological Abstract, Volume 82, No. 10, issued 15 November 1986, A. Robert; see page 96423, column 2

## Description

The present invention relates to the field of ophthalmology. More particularly, the invention relates to the treatment of glaucoma and associated elevations of intraocular pressure, and to the treatment of ocular hypertension associated with other diseases or conditions. The invention is directed to providing topical, ophthalmic, pharmaceutical compositions which include, as principal active ingredients, combinations of one or more beta-1 selective beta-blockers (e.g., betaxolol) and one or more sympathomimetic agents having beta-2 agonist activity (e.g., epinephrine).

The underlying causes of glaucoma are not fully understood. The symptomatology of this disease includes elevated pressure levels within the eye. These pressure elevations may be caused by either over production of fluid within the eye, or inadequate outflow of fluid from the eye. The intraocular fluid is referred to as "aqueous humor. The internal pressure of the eye associated with the amount of fluid inside the eye is referred to as "intraocular pressure" or "IOP. " Although the causes of glaucoma and associated elevations of intraocular pressure are not fully understood, the prognosis of untreated or inadequately treated glaucoma is known to include very serious ocular manifestations, namely blindness or significant loss of vision. Thus, there is a continuing need for therapies which control the elevated intraocular pressure associated with glaucoma.

A significant number of glaucoma patients are required to administer more than one drug in order to achieve therapeutic control of their intraocular pressure. In other words, a single drug does not provide adequate control of intraocular pressure in these patients. The drugs currently utilized in the treatment of glaucoma include miotics (e.g., pilocarpine, carbachol, and acetylcholinesterase inhibitors), sympathomimetics (e.g., epinephrine and dipivalylepinephrine), beta-blockers (e.g., betaxolol, levobunolol and timolol) and carbonic anhydrase inhibitors (e.g., acetazolamide, methazolamide and ethoxzolamide). Miotics and sympathomimetics are believed to lower intraocular pressure by increasing the outflow of aqueous humor, while beta-blockers and carbonic anhydrase inhibitors are believed to lower intraocular pressure by decreasing the formation of aqueous humor. All four types of drugs have potential side effects. Miotics, such as pilocarpine, can cause blurring of vision and other visual side effects which may either decrease patient compliance or require termination of miotic drug therapy. Carbonic anhydrase inhibitors can also cause serious side effects which affect patient compliance and/or necessitate withdrawal of the drug therapy. At least one beta-blocker, timolol, has increasingly become associated with serious pulmonary side effects attributable to its effect on beta-2 receptors in pulmonary tissue. In addition to these side effects, a therapy regimen which includes the use of two or more pharmaceutical compositions containing drugs selected from two or more of the above-cited classes requires the patient to apply the compositions to the affected eye(s) in separate, spaced dosages, several times per day. Patient compliance with such complicated dosage regimens can be very poor, particularly in elderly patients. Since the majority of glaucoma patients are elderly, this patient compliance problem is significant.

In light of the foregoing circumstances, it is clear that a need exists for new, more potent antiglaucoma compositions which avoid or reduce the above-cited side effects and enhance patient compliance. The present invention is directed to the provision of such compositions.

Sympathomimetic agents, which for purposes of the present specification are defined as compounds having beta-2 agonist activity, are known to lower intraocular pressure. Epinephrine and dipivalylepinephrine have been used for that purpose for many years. Sympathomimetic agents are believed to reduce intraocular pressure by increasing the outflow of aqueous humor through the trabecular meshwork of the eye. Although the mechanism is not entirely clear, it is believed that these agents promote the outflow of aqueous humor by binding with beta-2 receptors in the trabecular meshwork.

Beta-blockers, such as timolol and betaxolol, are also known to lower intraocular pressure, and have been utilized for that purpose for several years. The biological mechanism by which beta-blockers lower intraocular pressure is not entirely clear. It is believed that these compounds control intraocular pressure by decreasing the production of aqueous humor in the ciliary processes of the eye.

As mentioned above, two or more different types of drugs are sometimes required to achieve therapeutic control of intraocular pressure. The use of a drug which increases outflow of aqueous humor with a drug which reduces aqueous humor formation has the advantage of reducing intraocular pressure via two different mechanisms. Since two different mechanisms are involved, it might be expected that the reduction in intraocular pressure would be additive; that is, the total reduction in intraocular pressure achieved with the two drugs is the sum of the reductions achieved with each type of drug. However, previous studies have indicated that the concurrent use of epinephrine, a sympathomimetic compound, and timolol, a beta-blocker, does not result in such an additive reduction in intraocular pressure. Rather, the results of these studies suggest that timolol somehow interferes with the action of epinephrine. See Allen et al., "Additive Effect of Betaxolol and Epinephrine in Primary Open-Angle Glaucoma," Archives of Ophthalmology, Vol. 104, pages 1178-1184 (1986), and the references cited therein. The results of the study by Allen et al. also indicate that the concurrent use of a composition containing betaxolol and a composition containing epinephrine does result in an additive reduction of intraocular pressure.

The present invention is directed to topical, ophthalmic, pharmaceutical compositions containing combinations of certain beta-blocking agents and sympathomimetic agents, which compositions are surprisingly effective in lowering intraocular pressure. The beta-blockers utilized in the present invention are beta-1 selective. This means that the compounds bind predominantly with beta-1 receptors rather than beta-2 receptors. While applicant does not wish to be bound by any theory, it is believed that the beta-1 selective beta-blockers utilized in the present invention do not interfere significantly with the outflow-enhancing activity of sympathomimetic agents, because those beta-blockers bind predominantly with beta-1 receptors rather than beta-2 receptors, and therefore do not interfere or compete with the binding of sympathomimetic agents with the beta-2 receptors of the trabecular meshwork. Whatever the mechanism, it is clear that compositions containing a combination of a beta-1 selective beta-blocker, such as betaxolol, and a sympathomimetic agent, such as epinephrine, reduce intraocular pressure to an extent greater than that achievable by utilizing either agent alone.

According to a first aspect of the invention, there is provided a topical ophthalmic composition useful in controlling intraocular pressure, comprising a mixture of one or more sympathomimetic agents having β-2 agonist activity and one or more β-1 selective β-blockers, effective to control intraocular pressure, and a pharmaceutically acceptable carrier.

The ratio of the sympathomimetic component to the beta-1 selective beta-blocker component may vary considerably depending on the relative potency of the specific components utilized and other factors, such as the degree of intraocular pressure reduction desired and the nature of the condition being treated. With respect to the latter factor, it is conceivable that increasing aqueous humor outflow in some patients will have a more profound effect on intraocular pressure than reducing aqueous humor production, or vice versa. The ratio of the components employed is therefore left to the discretion of skilled clinicians. The ratio on a percent by weight concentration basis will typically be in the range of about 0.1:10 to 10:0.1 sympathomimetic agent:beta-1 selective beta-blocker.

The sympathomimetic agents which can be employed in the compositions of the present invention include all pharmaceutically acceptable compounds capable of enhancing the outflow of aqueous humor. In view of the apparent mechanism of action of the sympathomimetics, any pharmaceutically acceptable compound having beta-2 agonist activity may be utilized as the sympathomimetic component of the present compositions. Preferred sympathomimetic compounds include epinephrine, dipivalylepinephrine, norepinephrine, isoproterenol and the pivaloyloxy and phenylacetyloxy ester derivatives of epinephrine and norepinephrine described in U.S. Patents Nos. 3,809,714; 3,839,584: and 4,085,270. Epinephrine and dipivalylepinephrine are particularly preferred. The antiglaucoma compositions of the present invention will typically contain one or more sympathomimetic agents in an amount of about 0.02 to 2 0 percent by weight (wt. %).

The beta-1 selective beta-blockers which may be utilized in the present invention include all pharmaceutically acceptable compounds which are capable of reducing the production of aqueous humor and have a predominant affinity for beta-1 receptors rather than beta-2 receptors. The most preferred beta-1 selective beta-blocker is betaxolol. Other preferred beta-1 selective beta-blockers include acebutolol, adimolol, atenolol, befunolol, bisoprolol, carteolol, celiprolol, cetamolol, cicloprolol, draquinolol, epanolol, esmolol, metoprolol, SKF 95018, salcardolol, tienoxolol, tolamolol, and xamoterol. All of the foregoing compounds are known. The antiglaucoma compositions of the present invention will typically contain one or more beta-1 selective beta-blockers in an amount of about 0.01 to 3.0 percent by weight (wt. %).

In a second aspect, the present invention provides a topical ophthalmic composition, for controlling intraocular pressure, comprising: a mixture of 0.02 to 2.0 percent by weight of a β-2 agonist and 0.01 to 3.0 percent by weight of betaxolol; and a pharmaceutically acceptable vehicle therefor. Preferably, the β-2 agonist is selected from the group consisting of epinephrine, norepinephrine, dipivalylepinephrine, isoproterenol, pivaloyloxy and phenylacetyloxy esters of epinephrine, and pivaloyloxy and phenylacetyloxy esters of norepinephrine and the ratio of the β-2 agonist to the betaxolol, on a percent by weight concentration basis, is in the range of 0.1:10 to 10:0.1.

In further aspects, the invention extends to the use of compositions in accordance with the first or second aspects of the invention, in the manufacture of topical ophthalmic compositions for use in controlling intraocular pressure.

In addition to the above-described principal active ingredients, the antiglaucoma compositions of the present invention may further comprise various formulatory ingredients, such as antimicrobial preservatives and tonicity agents. Examples of suitable antimicrobial preservatives include: benzalkonium chloride, thimerosal, chlorobutanol, methyl paraben, propyl paraben, phenylethyl alcohol, edetate disodium, sorbic acid, ONAMER M and other agents equally well known to those skilled in the art. Such preservatives, if utilized, will typically be employed in an amount of from about 0.001% to 1.0% by weight (wt. %). Examples of suitable agents which may be utilized to adjust the tonicity or osmolality of the formulations include sodium chloride, potassium chloride, mannitol, dextrose, glycerine and propylene glycol. Such agents, if utilized, will be employed in an amount of about 0.1% to 10.0% by weight (wt. %).

As will be appreciated by those skilled in the art, the compositions may be formulated in various dosage forms suitable for topical ophthalmic delivery, including solutions, suspensions, emulsions, gels, and erodible solid ocular inserts. The compositions are preferably aqueous, and have a pH in the range of 3.5 to 8.0 and an osmolality in the range of 280 to 320 millimoles per liter (mm/l).

The following example further illustrates the antiglaucoma compositions of the present invention.

### Example

The following formulation is typical of aqueous ophthalmic solutions of the present invention.

| Ingredient | Amount (wt. %) |
|---|---|
| Dipivalylepinephrine | 0.1 |
| Betaxolol | 0.5 |
| Benzalkonium Chloride | 0.01 |
| Edetate Sodium | 0.05 |
| HCl or NaOH | QS pH 7.4 |
| Purified Water | QS 100 |

The compositions of the present invention are useful in methods of treating and controlling ocular hypertension associated with glaucoma and other ophthalmic diseases and abnormalities. The methods comprise topical application of a therapeutically effective amount of a composition according to the present invention to the affected eye(s) of the patient. The frequency and amount of the dosage will be determined by the clinician based on various clinical factors. The methods will typically comprise topical application of one to two drops (or an equivalent amount of a solid or semisolid dosage form) to the affected eye one to two times per day.

## Claims

1. A topical ophthalmic composition useful in controlling intraocular pressure, comprising a mixture of one or more sympathomimetic agents having β-2 agonist activity and one or more β-1 selective β-blockers, effective to control intraocular pressure, and a pharmaceutically acceptable carrier.

2. A topical ophthalmic composition according to claim 1, wherein the β-2 agonist is selected from epinephrine, norepinephrine, isoproterenol, and pivaloyloxy and phenylacetyloxy esters of epinephrine and norepinephrine, and the β-1 selective β-blocker is selected from acebutolol, adimolol, atenolol, befunolol, betaxolol, bisoprolol, carteolol, celiprolol, cetamolol, cicloprolol, draquinolol, epanolol, esmolol, metoprolol, SKF 95018, salcardolol, tienoxolol, tolamolol, and xamoterol.

3. A topical ophthalmic composition according to claim 1, wherein the β-2 agonist comprises epinephrine and the β-1 selective β-blocker comprises betaxolol.

4. A topical ophthalmic composition according to claim 1, wherein the β-2 agonist comprises dipivalylepinephrine and the β-1 selective β-blocker comprises betaxolol.

5. Use of a combination of one or more sympathomimetic agents having β-2 agonist activity with one or more β-1 selective β-blockers, in the manufacture of a topical ophthalmic composition as claimed in any of claims 1-4, for use in controlling intraocular pressure.

6. A topical ophthalmic composition, for controlling intraocular pressure, comprising: a mixture of 0.02 to 2.0 percent by weight of a sympathomimetic agent having β-2 agonist activity and 0.01 to 3.0 percent by weight of a β-1 selective β-blocker comprising betaxolol; and a pharmaceutically acceptable vehicle therefor.

7. A topical ophthalmic composition, as claimed in claim 6, wherein the β-agonist is is selected from the group consisting of epinephrine, norepinephrine, dipivalylepinephrine, isoproterenol, pivaloyloxy and phenylacetyloxy esters of epinephrine, and pivaloyloxy and phenylacetyloxy esters of norepinephrine and the ratio of the β-2 agonist to the betaxolol, on a percent by weight concentration basis, is in the range of 0.1:10 to 10:0.1.

8. A composition according to claim 6 or claim 7, wherein the β-2 agonist is selected from the group consisting of epinephrine and dipivalylepinephrine.

9. A composition according to claim 8, wherein the β-2 agonist comprises dipivalylepinephrine.

10. Use of a mixture for the manufacture of a medicament for controlling intraocular pressure, which mixture comprises a therapeutically effective amount of a composition comprising: 0.02 to 2.0 percent by weight of a sympathomimetic agent having β-2 agonist activity and 0.01 to 3.0 percent by weight of a β-1 selective β-blocker comprising betaxolol; and a pharmaceutically acceptable vehicle therefor.

11. A use as claimed in claim 10, wherein the β-2 agonist is is selected from the group consisting of epinephrine, norepinephrine, dipivalylepinephrine, isoproterenol, pivaloyloxy and phenylacetyloxy esters of epinephrine, and pivaloyloxy and phenylacetyloxy esters of norepinephrine and the ratio of the β-2 agonist to the betaxolol, on a percent by weight concentration basis, is in the range of 0.1:10 to 10:0.1.

12. A use as claimed in claim 10 or claim 11, wherein the β-2 agonist is selected from the group consisting of epinephrine and dipivalylepinephrine.

13. A use as claimed in any of claims 10-12, wherein the β-2 agonist comprises dipivalylepinephrine.

14. A topical ophthalmic composition, as claimed in claim 1, comprising betaxolol as a β-1 selective β-blocker.

## Patentansprüche

1. Topische ophthalmologische Zusammensetzung, verwendbar bei der Regelung des intraokularen Druckes, umfassend eine Mischung aus einem oder mehreren sympathomimetischen Mitteln mit Beta-2-Agonisten-Aktivität und einem oder mehreren Beta-1-selektiven Beta-Blockern, welche zur Regelung des intraokularen Druckes wirksam sind, und einen pharmazeutisch annehmbaren Träger.

2. Topische ophthalmologische Zusammensetzung nach Anspruch 1, worin der Beta-2-Agonist ausgewählt ist unter Epinephrin, Norepinephrin, Isoproterenol und Pivaloyloxy- und Phenylacetyloxyestern von Epinephrin und Norepinephrin und der Beta-1-selektive Beta-Blocker ausgewählt ist unter Acebutolol, Adimolol, Atenolol, Befunolol, Betaxolol, Bisoprolol, Carteolol, Celiprolol, Cetamolol, Cicloprolol, Draquinolol, Epanolol, Esmolol, Metoprolol, SKF 95018, Salcardolol, Tienoxolol, Tolamolol und Xamoterol.

3. Topische ophthalmologische Zusammensetzung nach Anspruch 1, worin der Beta-2-Agonist Epinephrin umfaßt und der Beta-1-selektive Beta-Blocker Betaxolol umfaßt.

4. Topische ophthalmologische Zusammensetzung nach Anspruch 1, worin der Beta-2-Agonist Dipivalylepinephrin umfaßt und der Beta-1-selektive Beta-Blocker Betaxolol umfaßt.

5. Verwendung einer Kombination aus einem oder mehreren sympathomimetischen Mitteln mit Beta-2-Agonisten-Aktivität und einem oder mehreren Beta-1-selektiven Beta-Blockern bei der Herstellung einer topischen ophthalmologischen Zusammensetzung nach irgendeinem der Ansprüche 1-4 zur Verwendung bei der Regelung des intraokularen Druckes.

6. Topische ophthalmologische Zusammensetzung zur Regelung des intraokularen Druckes umfassend: eine Mischung aus 0,02 bis 2,0 Gew.-% eines sympathomimetischen Mittels mit Beta-2-Agonisten-Aktivität und 0,01 bis 3 Gew.-% eines Beta-1-selektiven Betaxolol umfassenden Beta-Blockers; und ein pharmazeutisch annehmbares Vehikel dafür.

7. Topische ophthalmologische Zusammensetzung nach Anspruch 6, worin der Beta-Agonist ausgewählt ist aus der Gruppe bestehend aus Epinephrin, Norepinephrin, Dipivalylepinephrin, Isoproterenol, Pivaloyloxy- und Phenylacetyloxyestern von Epinephrin und Pivaloyloxy- und Phenylacetyloxyestern von Norepinephrin und das Verhältnis des Beta-2-Agonisten zum Betaxolol, auf Gewichtsprozent-Konzentrationsbasis, im Bereich von 0,1 : 10 bis 10 : 0,1 liegt.

8. Zusammensetzung nach Anspruch 6 oder 7, worin der Beta-2-Agonist aus der aus Epinephrin und Dipivalylepinephrin bestehenden Gruppe ausgewählt ist.

9. Zusammensetzung nach Anspruch 8, worin der Beta-2-Agonist Dipivalylepinephrin umfaßt.

10. Verwendung einer Mischung zur Herstellung eines Medikaments zur Regelung des intraokularen Druckes, wobei die Mischung eine therapeutisch wirksame Menge einer: 0,02 bis 2,0 Gew.-% eines sympathomimetischen Mittels mit Beta-2-Agonisten-Aktivität und 0,01 bis 3,0 Gew.-% eines Beta-1-selektiven, Betaxolol umfassenden Beta-Blockers umfassenden Zusammensetzung; und ein pharmazeutisch annehmbares Vehikel dafür umfaßt.

11. Verwendung nach Anspruch 10, worin der Beta-2-Agonist ausgewählt ist aus der aus Epinephrin, Norepinephrin, Dipivalylepinephrin, Isoproterenol, Pivaloyloxy- und Phenylacetyloxyestern von Epinephrin und Pivaloyloxy- und Phenylacetyloxyestern von Norepinephrin bestehenden Gruppe und das Verhältnis des Beta-2-Agonisten zum Betaxolol, auf Gewichtsprozent-Konzentrationsbasis, im Bereich von 0,1 : 10 bis 10 : 0,1 liegt.

12. Verwendung nach Anspruch 10 oder Anspruch 11, worin der Beta-2-Agonist ausgewählt ist aus der aus Epinephrin und Dipivalylepinephrin bestehenden Gruppe.

13. Verwendung nach irgendeinem der Anprüche 10-12, worin der Beta-2-Agonist Dipivalylepinephrin umfaßt.

14. Topische ophthalmologische Zusammensetzung nach Anspruch 1, umfassend Betaxolol als einen Beta-1-selektiven Beta-Blocker.

## Revendications

1. Composition ophtalmique topique utile pour le contrôle de la pression intra-oculaire, comprenant un mélange d'un ou plusieurs agent sympathomimétiques ayant une activité β-2-agoniste et d'un ou plusieurs β-bloquants β-1-sélectifs, efficace pour le contrôle de la pression intra-oculaire, et d'un véhicule pharmaceutiquement acceptable.

2. Composition ophtalmique topique selon la revendication 1, dans laquelle le β-2-agoniste est choisi entre l'épinéphrine, la norépinéphrine, l'isoprotérénol et les pivaloyloxy- et phénylacétyloxy-esters de l'épinéphrine et de la norépinéphrine, et le β-bloquant β-1-sélectif est choisi entre l'acébutolol, l'adimolol, l'aténolol, le béfunolol, le bétaxolol, le bisoprolol, le cartéolol, le celiprolol, le cétamolol, le cicloprolol, le draquinolol, l'épanolol, l'esmolol, le métoprolol, le SKF 95018, le salcardolol, le tienoxolol, le tolamolol et le xamotérol.

3. Composition ophtalmique topique selon la revendication 1, dans laquelle le β-2-agoniste est l'épinéphrine et le β-bloquant β-1-sélectif est le bétaxolol.

4. Composition ophtalmique topique selon la revendication 1, dans laquelle le β-2-agoniste est la dipivalylépinéphrine et le β-bloquant β-1-sélectif est le bétaxolol.

5. Utilisation d'une combinaison d'un ou plusieurs agents sympathomimétiques ayant une activité β-2-agoniste avec un ou plusieurs β-bloquants β-1-sélectifs, dans la fabrication d'une composition ophtalmique topique selon l'une quelconque des revendications 1 à 4, destinée au contrôle de la pression intra-oculaire.

6. Composition ophtalmique topique pour le contrôle de la pression intra-oculaire, comprenant un mélange de 0,02 à 2,0 pour cent en poids d'un agent sympathomimétique ayant une activité β-2-agoniste et 0,01 à 3,0 pour cent en poids d'un β-bloquant β-1-sélectif consistant en bétaxolol et un véhicule pharmaceutiquement acceptable.

7. Composition ophtalmique topique selon la revendication 6, dans laquelle le β-agoniste est choisi entre l'épinéphrine, la norépinéphrine, la dipivalylépinéphrine, l'isoprotérénol, les pivaloyloxy- et phénylacétyloxy-esters de l'épinéphrine, et les pivaloyloxy- et phénylacétyloxy-esters de la norépinéphrine, et la proportion entre le β-2-agoniste et le bétaxolol, sur la base du pourcentage en poids des concentrations est comprise entre 0,1 : 10 et 10 : 0,1.

8. Composition selon la revendication 6 ou la revendication 7, dans laquelle le β-2-agoniste est choisi entre l'épinéphrine et la dipivalylépinéphrine.

9. Composition selon la revendication 8, dans laquelle le β-2-agoniste est la dipivalylépinéphrine.

10. Utilisation d'un mélange pour la fabrication d'un médicament pour le contrôle de la pression intra-oculaire, ce mélange comprenant une quantité thérapeutiquement efficace d'une composition comprenant 0,02 à 2,0 pour cent en poids d'un agent sympathomimétique ayant une activité β-2-agoniste et 0,01 à 3,0 pour cent en poids d'un β-bloquant β-1-sélectif consistant en bétaxolol et un véhicule pharmaceutiquement acceptable.

11. Utilisation selon la revendication 10, dans laquelle le β-2-agoniste est choisi entre l'épinéphrine, la norépinéprine, la dipivalylépinéphrine, l'isoprotérénol, les pivaloyloxy- et phénylacétyloxy-esters de l'épinéphrine et les pivaloyloxy- et phénylacétyloxy-esters de la norépinéphrine, et la proportion entre le β-2-agoniste et le bétaxolol, sur la base du pourcentage en poids des concentrations, est comprise entre 0,1 : 10 et 10 : 0,1.

12. Utilisation selon la revendication 10 ou la revendication 11, dans laquelle le β-2-agoniste est choisi entre l'épinéphrine et la dipivalylépinéphrine.

13. Utilisation selon l'une quelconque des revendications 10 à 12, dans laquelle le β-2-agoniste est la dipivalylépinéphrine.

14. Composition ophtalmique topique selon la revendication 1, comprenant du bétaxolol comme β-bloquant β-1-sélectif.
